# EUROPEAN PATENT APPLICATION

(11) **EP 4 722 828 A1**
(43) Date of publication of application: **08.04.2026**
(21) Application number: 24204110.1
(22) Date of filing: 02.10.2024
(51) Int. Cl.: G05B 13/04, B01J 19/00, C07C 273/04

(54) **METHOD FOR CONTROLLING A COMPOSITION VARIABLE IN A UREA-PRODUCING PROCESS**

(71) Applicant: Yara International ASA, 0277 Oslo (NO)
(72) Inventor: Dorini, Francesco, 44122 Ferrara (IT); Padovani, Andrea, 44123 Ferrara (IT); Porro, Lino Giovanni, 1040 Etterbeek (BE); Sozzi, Alessio, 44123 Ferrara (IT)
(74) Representative: AWA Sweden AB

(57) **Abstract**

The present disclosure provides a virtual sensing method for controlling at least one composition variable during a urea production process in a urea-producing plant, based on a plurality of online measured process variables and the viscosity of one or more liquid streams.

## Description

### Field of the disclosure

The present disclosure is related to the field of urea production.

### Background information

Urea is an important chemical product which is mainly utilized as fertilizer.

Different types of processes for the production of urea exist, such as the Stamicarbon, Saipem, Casale, and Toyo processes. All these processes start with the reaction between ammonia and carbon dioxide at high pressure (e.g., 13-30 MPa) and high temperature (e.g., 180-200° C) in a reactor. An aqueous solution comprising water, urea, ammonium carbamate and free ammonia, also called the synthesis solution, is obtained and gradually transformed into a urea melt, a composition comprising at least 95.0 weight% of urea, and less than 5.0 weight% of water. The transformation of the synthesis solution into a urea melt generates waste streams comprising ammonia and carbon dioxide that are re-injected in the synthesis section to increase the yield of the process.

The reaction between ammonia and carbon dioxide to form urea in the reactor is an equilibrium, so the chemical composition of the synthesis solution produced therein is highly dependent on the reaction conditions, in particular the amounts of carbon dioxide, ammonia, and water. Rather than by the exact amounts of these compounds, process information is carried by the ratios of these compounds. In a urea-production process, it is conventional to refer to these ratios as the NH₃/CO₂ ratio, which is the molar ratio between the total nitrogen content of a composition (as ammonia, urea, and ammonium salts) and the total carbon content (as free carbon dioxide, urea, or other salts) of the same composition, and the H₂O/CO₂ ratio, which is the molar ratio between the water content of a composition and the total carbon content of the same composition.

For a plant operator, it is very important to maintain these ratios as stable as possible around a desired target value to increase the stability of the plant, maintain a high production rate, and reduce its energy and raw material consumption.

It is possible to measure these ratios using particular analytical methods, but these require expensive equipment, such as a Raman spectrometer. As an alternative, mathematical models have been developed to evaluate the NH₃/CO₂ and H₂O/CO₂ ratios in real time. These models use a large number of process variables that can be accurately measured to estimate the value of the NH₃/CO₂ and H₂O/CO₂ ratios of the plant. Although these models have greatly increased the reliability of urea-producing plants, there is a need to provide more precise models which can further improve operations.

WO2019149937A1 (Yara International, 2019) discloses a virtual sensing method and system for controlling at least one composition variable in a urea production process, based on a plurality of online measured process variables and a model, wherein the model is used to estimate, during the urea production process, the at least one composition variable indicative of a urea content on the basis of the plurality of online measured process variables, and modifying at least one of the plurality of online measured process variables for ensuring that a value of the at least one composition variable is within a predetermined range.

### Summary of the disclosure

In the method of the present disclosure, the viscosity of liquid streams of various sections of a urea-producing plant is taken into consideration to produce a model for estimating the NH₃/CO₂ and H₂O/CO₂ ratios of a composition in the plant. The model has a higher precision that previously known models, and the model is used to modify and/or control a process variable of the production process.

In a first aspect, the present disclosure provides a virtual sensing method for controlling at least one composition variable during a urea production process in a urea-producing plant, based on a plurality of online measured process variables and the viscosity of one or more liquid streams, and a model, wherein the model is used to estimate, during the urea production process, the at least one composition variable, on the basis of the plurality of online measured process variables and the viscosity of one or more liquid streams, wherein the method includes modifying at least one of the plurality of online measured process variables for ensuring that the value of the at least one composition variable is within a predetermined range, wherein the model is obtained by: retrieving, over a first period of time during the urea production process, a plurality of online measurement data relating to a plurality of predetermined process variables and the viscosity of one or more liquid streams by means of a plurality of sensors arranged in the urea-producing plant, the plurality of predetermined process variables comprising at least one of the group consisting of a flow rate, a liquid level, a temperature, and a pressure; retrieving, at time points within the first period of time, a plurality of offline measurement data of the at least one composition variable; and processing the plurality of online and offline measurement data and performing a statistical analysis for identifying the model for predicting the at least one composition variable on the basis of the plurality of predetermined process variables.

In another aspect, the present disclosure provides a method for obtaining a model for controlling at least one composition variable in a urea production process in a urea-producing plant, the method comprising:
- retrieving, over a first period of time during the urea production process, a plurality of online measurement data relating to a plurality of predetermined process variables and to the viscosity of a liquid stream by means of a plurality of sensors arranged in the urea-producing plant, the plurality of predetermined process variables comprising at least one from the group consisting of a flow rate, a liquid level, a temperature, and a pressure;
- retrieving, at time points within the first period of time, a plurality of offline measurement data of at least one composition variable; and
- processing the plurality of online and offline measurement data and performing a statistical analysis for identifying the model for predicting the at least one composition variable on the basis of the plurality of predetermined process variables and the viscosity.

### Brief description of the figure

The following description of the figure of a specific embodiment of a system according to the present disclosure is only given by way of example and is not intended to limit the present explanation, its application or use. In the figure, identical reference numerals refer to the same or similar parts and features.

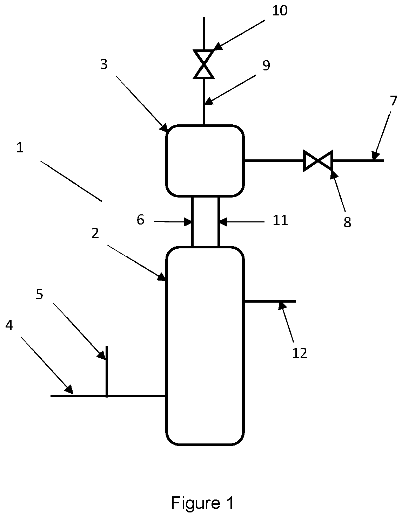

Figure 1 shows a system to which an embodiment of a virtual sensing method according to the first aspect can be applied.

### Detailed description of the disclosure

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g., component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "weight percent", "%wt" or "weight%", here and throughout the description unless otherwise defined, refers to the relative weight of the respective component based on the overall weight of the formulation.

In a first aspect, the present disclosure provides a virtual sensing method for controlling at least one composition variable during a urea production process in a urea-producing plant, based on a plurality of online measured process variables and the viscosity of one or more liquid streams, and a model,
wherein the model is used to estimate, during the urea production process, the at least one composition variable, on the basis of the plurality of online measured process variables and the viscosity of one or more liquid streams, wherein the method includes modifying at least one of the plurality of online measured process variables for ensuring that the value of the at least one composition variable is within a predetermined range,
wherein the model is obtainable or obtained by:
   retrieving, over a first period of time during the urea production process, a plurality of online measurement data relating to a plurality of predetermined process variables and the viscosity of one or more liquid streams by means of a plurality of sensors arranged in the urea-producing plant, the plurality of predetermined process variables comprising at least one of the group consisting of a flow rate, a liquid level, a temperature, and a pressure;
   retrieving, at time points within the first period of time, a plurality of offline measurement data of the at least one composition variable; and
   processing the plurality of online and offline measurement data and per forming a statistical analysis for identifying the model for predicting the at least one composition variable on the basis of the plurality of predetermined process variables.

The at least one composition variable in the urea production process can be controlled on the basis of the data measured online and the obtained model. It will be appreciated that the model can be obtained or determined separately from the method of controlling the urea production process. For instance, the model can be already pre-determined and used for controlling process parameters of the urea production process. The online measured data can be obtained directly from the process (without laboratory intervention). The online measured data can be obtained in real time.

It has been found that composition variables in a urea production process vary with the viscosity of the compositions produced during the production process. By incorporating viscosity as a parameter in a model, it is possible to estimate the value of a composition variable in the urea production process with higher accuracy.

The viscosity of a fluid is a measure of its resistance to deformation at a given rate. In chemical manufacturing, two types of viscosity can be measured: the dynamic viscosity, or the kinetic viscosity; the kinetic viscosity being equal to the ratio of the dynamic viscosity over the density of the fluid. Both types of viscosities may be used in the model.

There are different types of sensors for measuring the viscosity of a fluid in a chemical production process. For example, the viscosity sensor may comprise an element, such as a bar, that is configured to impose a torsional movement on the fluid, and the sensor comprises elements that can detect the velocity profile of the fluid across a distance. Alternatively, the viscosity sensor may be a resonance frequency meter, which is configured to measure the vibrations created by the flow of a liquid on a probe.

However, the viscosity alone is not enough to estimate a composition variable of the production process, it is necessary to also take process variables into consideration to build a reliable model.

In some embodiments, by using the model, measuring composition variables, such as NH₃/CO₂ and H₂O/CO₂ ratios in the urea-producing plant by sample analysis may no longer be necessary. Hence, it may no longer be required to manually take samples, often involving increased safety risks, for performing offline experiments in a lab. Consequently, less time and resources are needed for controlling the urea production process by means of the model. In this way, the costs involved can be reduced, since such measurement units are typically expensive, require a great deal of maintenance and/or are often difficult to implement in an existing urea-producing plant.

Process variables, such as, e.g., temperature, flow, and / or pressure, as well as some composition variables, such as a fluid density and a fluid viscosity, may be very easily measured online, i.e., during the operation of the plant. On the contrary, some composition variables, such as NH₃/CO₂ and H₂O/CO₂ ratios of a fluid or composition, cannot be measured online.

The model comprises a mathematical model, configured to use a number of process variables, in the form of a plurality of online measured process variables and of at least one viscosity, which affect the at least one composition variable in the urea production process, such as the NH₃/CO₂ and H₂O/CO₂ ratios of a liquid composition. In an advantageous way, using the model, the NH₃/CO₂ and H₂O/CO₂ ratios can be predicted without needing to wait for the lab analysis. Hence, the control of the urea production process can be enhanced, so that also the efficiency of the urea production process can be improved. The model may be determined for a high-pressure part, a medium-pressure part, or a low-pressure part of a urea-producing plant.

The method can thus be used for controlling an operation of a urea-producing plant. Operators in a urea-producing plant can act on different parameters to modify the operating conditions of a plant, for example increasing or decreasing the input of a component, such as raw material, steam, demineralized water, cooling water, and electricity, closing or opening a valve controlling the flow of a fluid into the process. Modifying the at least one process variable can include adjusting a current process variable in the urea production process based on the predicted at least one composition variable using the model.

The urea production process can be controlled towards a stable steady-state operation by means of the model. In some embodiments, a statistical method is employed for obtaining a linear steady-state model for predicting the at least one composition variable. The NH₃/CO₂ and/or H₂O/CO₂ ratios of a fluid at a certain stage in the urea production process can be chosen as the composition variable in the urea production process.

In some embodiments, the plurality of online measured process variables and the viscosity obtained by means of online measurements over a second period of time, different from the first period of time, are provided as inputs to the identified model, wherein the model provides as an output at least one predicted composition variable, which is being controlled.

The model can be obtained or determined in advance, i.e., using online and offline measurement data obtained during the first time period. The first time period can also be considered as a model building period, in which sufficient data is gathered over a period of time (e.g., weeks, months) to build the model. When the model has been obtained or determined, it can be used for predicting the composition variable based on the online measured process variables provided as input. In this way, the model can determine which configuration of process variables are to be used to obtain an optimum (model predicted) composition variable. In some embodiments, the model can be updated continuously by performing (less frequent) offline measurements. More measurement points may improve the accuracy of the model, compensate for changes in the urea synthesis plant and/or changes in ambient conditions.

In some embodiments, gathered sensor data from online measurements are stored in a data store, wherein a reduced data set is obtained from the data store, wherein the model is identified based on the reduced data set, the model providing a correlation between the reduced data set and the at least one composition variable.

The plurality of online measured process variables can be sampled down in different ways, for instance by averaging over time (e.g., over 5-minute time intervals). In some embodiments, a computer program product is used which is configured to collect, optionally arrange, and down-sample large quantities of available raw plant data relating to the plurality of online measured process variables and the viscosity of one or more liquid streams, which are measured in high resolution by means of the plurality of sensors.

In some embodiments, the composition variable is selected from the group consisting of a NH₃/CO₂ ratio and a H₂O/CO₂ ratio. The NH₃/CO₂ ratio is defined as a ratio between a total NH₃ content and a total CO₂ content of a stream, in particular an aqueous stream, of the plant. The H₂O/CO₂ ratio is defined as a ratio between a total H₂O content and a total CO₂ content of a stream, in particular an aqueous stream, of the plant. The NH₃/CO₂ and/or H₂O/CO₂ ratios of a liquid composition recovered at the outlet of a carbamate condenser are important data in a urea-producing plant because the liquid composition is eventually re-injected in the urea reactor. Therefore, it is important to know and control one, or both, ratios to ensure optimum performance of the synthesis reaction and of the plant. In particular, the operator can increase or decrease the amount of water injected in a carbamate decomposer based on the values of one or both ratios predicted by the model.

In some embodiments, the composition variable is selected from the group consisting of a NH₃/CO₂ ratio defined as a ratio between a total equivalent NH₃ and a total equivalent CO₂, a H₂O/CO₂ ratio defined as a ratio between a total equivalent H₂O and a total equivalent CO₂, and/or an extent of reaction (or carbon dioxide conversion) defined as a ratio between urea and total equivalent CO₂.

In some embodiments, the process variables comprise at least one selected from the group consisting of a plant load, a CO₂ feed flow, a CO₂ flow to a CO₂ stripper or to a reactor, a passivation air flow to a reactor, a passivation air flow to a stripper, a carbamate recycle flow to a carbamate condenser, a carbamate recycle flow to the high-pressure scrubber, a steam flow from carbamate condenser, a total flow of NH3, a flow of NH3 to a carbamate condenser, a flow of NH3 to a carbamate ejector, a flow of NH3 to a reactor, a steam consumption of a stripper, a pressure in a reactor, a carbamate condenser steam pressure, a stripper vapor exit temperature, a stripper liquid exit temperature, a temperature of an NH3 feed, a temperature of a stream comprising ammonium carbamate, a temperature in a reactor, a temperature of a urea solution from a reactor, a temperature at a liquid outlet of a stripper, a temperature at the liquid outlet of a carbamate condenser, a pressure difference in a urea reactor outlet valve, a liquid level in a reactor, a liquid level in a scrubber, a flow of inert gas to a condenser, an opening degree of a pressure valve, and a liquid level in a liquid/vapor separator.

In some embodiments, the model is used to estimate, during the urea production process, the at least one composition variable, on the basis of the plurality of online measured process variables, the viscosity of one or more liquid streams, and the density of one or more liquid streams.

In some embodiments, the model is obtained by retrieving, over a first period of time during the urea production process, a plurality of online measurement data relating to a plurality of predetermined process variables, the viscosity of one or more liquid streams, and the density of one or more liquid streams, by means of a plurality of sensors arranged in the urea-producing plant. The plurality of predetermined process variables comprising at least one of the group consisting of a flow rate, a liquid level, a temperature, and a pressure. It has been found that, in some cases, a model incorporating the density and viscosity of one or more compositions was able to achieve an even higher reliability, meaning that the value of one or more composition variables predicted by the model are closer to the actual values of the one or more compositions variables measured offline. However, a viscosity and a density alone are not enough to estimate a composition variable of the production process, it is necessary to also take process variables into consideration to build a reliable model.

In some embodiments, a set of 2 to 10 variables, including process variables and at least one viscosity, is used for obtaining the model. In this way, over-optimization and dependency on a large number of online measurement devices can be avoided. In some embodiments, a set of 2, 3, 4, 5, 6, 7, 8, 9, or 10 process variables is used.

In some embodiments, a set of 8 to 15 variables, including process variables and at least one viscosity, is used for identifying the model. The number of variables used for identifying the suitable model may depend on the composition variable that the model is trying to predict, and/or the location in the plant of the composition that the model is trying to predict.

In some embodiments, the plurality of online measured process variables comprises a plant load, a flow of inert gas to a condenser, an opening degree of a pressure valve, a temperature at an inlet of a carbamate condenser, a pressure in a carbamate condenser, a temperature in a carbamate condenser, a pressure at the outlet of an absorber, and a density of the carbamate from the carbamate condenser. The NH₃/CO₂ and/or H₂O/CO₂ ratios of a liquid composition recovered at the outlet of a carbamate condenser are important data in a urea-producing plant because the liquid composition is eventually re-injected in the urea reactor, so it is important to know and control these ratios to ensure optimum performance of the synthesis reaction and thereby of the plant. It was found that a combination of process variables, including a plant load, a flow of inert gas to a condenser, an opening degree of a pressure valve, a temperature at an inlet of a carbamate condenser, a pressure in a carbamate condenser, a temperature in a carbamate condenser, a pressure at the outlet of an absorber, and a density of the carbamate from the carbamate condenser, combined with the viscosity of the liquid composition at the outlet of the carbamate condenser could be used to generate a precise model to predict these ratios.

In some embodiments, the statistical analysis comprises an algorithm for performing a principal component analysis or a partial least squares analysis. Such algorithms have been proven to provide a reliable model of composition variables in a urea-producing plant. Furthermore, they require less computing power compared to more advanced algorithms.

In some embodiments, the algorithm is an orthogonal partial least squares algorithm. An orthogonal partial least squares regression method (OPLS) can be used for obtaining a linear steady-state model for predicting the at least one composition variable. The model may be regarded as an empirical model of the urea-producing plant. Other techniques such as a correlation analysis, or a multivariate calibration are also possible for obtaining the steady-state model. It is appreciated that the statistical analysis may also include a machine learning algorithm, such as a neural network (learning) algorithm.

In some embodiments, the urea production process comprises a CO₂-stripping process, a self-stripping process, or a thermal stripping process, such as an isobaric double recycle process. Modern urea production processes comprise a stripping process, wherein the solution produced by the reactor is processed in a stripper to remove some of the ammonium carbamate comprised in the solution. This allows the plant to recover some energy via the condensation of the vapors of the stripper. Different stripping processes are available today, such as CO₂-stripping process, wherein a gas stream comprising CO₂ is injected into the stripper, and a thermal stripping process, which does not use a stripping gas.

In another aspect, the present disclosure provides a method for obtaining a model for controlling at least one composition variable in a urea production process in a urea-producing plant, the method comprising:
- retrieving, over a first period of time during the urea production process, a plurality of online measurement data relating to a plurality of predetermined process variables and to the viscosity of a liquid stream by means of a plurality of sensors arranged in the urea-producing plant, the plurality of predetermined process variables comprising at least one from the group consisting of a flow rate, a liquid level, a temperature, and a pressure;
- retrieving, at time points within the first period of time, a plurality of offline measurement data of at least one composition variable; and
- processing the plurality of online and offline measurement data and performing a statistical analysis for identifying the model for predicting the at least one composition variable on the basis of the plurality of predetermined process variables and the viscosity.

In some embodiments, the method for obtaining a model further comprises performing simulated annealing for identifying a selection of variables used with the model, wherein the simulated annealing comprises determining a plurality of variable sets with different combinations of process variables and viscosities, and evaluating, for each of the plurality of the variable sets, a quality of prediction of the at least one composition variable, wherein the variable set providing the highest quality of prediction of the at least one composition variable is selected for use with the model.

In some embodiments, the model is a mathematical linear steady-state model having a plurality of coefficients linked to a plurality of process variables and at least one viscosity.

In some embodiments, the density of a liquid is also retrieved over the first period of time during the urea production process, and the statistical analysis for identifying the model for predicting the at least one composition variable is performed on the basis of the plurality of predetermined process variables, the viscosity and the density.

In another aspect, the present disclosure provides a system including a controller configured for controlling at least one composition variable in a urea production process in a urea-producing plant, based on a plurality of online measured process variables and the viscosity of one or more liquid streams, and a model, wherein the controller is configured to use the model for estimating, during the urea production process, the at least one composition variable on the basis of the plurality of online measured process variables and the viscosity of one or more liquid streams, for ensuring that the value of the at least one composition variable is within a predetermined range, wherein the model is obtainable or obtained by:
retrieving, over a first period of time during the urea production process, a plurality of online measurement data relating to a plurality of predetermined process variables and the viscosity of one or more liquid streams by means of a plurality of sensors arranged in the urea-producing plant, the plurality of predetermined process variables comprising at least one of the group consisting of a flow rate, a liquid level, a temperature, and a pressure;
retrieving, at a time point within the first period of time, a plurality of offline measurement data of the at least one composition variable; and
processing the plurality of online and offline measurement data and performing a statistical analysis for obtaining the model for predicting the at least one composition variable on the basis of the plurality of predetermined process variables and the viscosity of one or more liquid streams.

In another aspect, the present disclosure provides a system for obtaining a model for controlling at least one composition variable in a urea production process, the system comprising a controller configured to perform:
- retrieving, over a first period of time during the urea production process, a plurality of online measurement data relating to a plurality of predetermined process variables and the viscosity of one or more liquid streams, by means of a plurality of sensors arranged in the urea-producing plant, the plurality of predetermined process variables comprising at least one of the group consisting of a flow rate, a liquid level, a temperature, and a pressure;
- retrieving, at time points within the first period of time, a plurality of offline measurement data of the at least one composition variable; and
- processing the plurality of online and offline measurement data and performing a statistical analysis for identifying the model for predicting the at least one composition variable on the basis of the plurality of predetermined process variables and the viscosity of one or more liquid streams.
In some embodiments, the controller comprised in the system is configured to perform:
- retrieving, over a first period of time during the urea production process, a plurality of online measurement data relating to a plurality of predetermined process variables, the viscosity of one or more liquid streams, and the density of one or more liquid streams by means of a plurality of sensors arranged in the urea-producing plant, the plurality of predetermined process variables comprising at least one of the group consisting of a flow rate, a liquid level, a temperature, and a pressure;
- retrieving, at time points within the first period of time, a plurality of offline measurement data of the at least one composition variable; and
- processing the plurality of online and offline measurement data and performing a statistical analysis for identifying the model for predicting the at least one composition variable on the basis of the plurality of predetermined process variables, the viscosity of one or more liquid streams, and the density of one or more liquid streams.

In another aspect, the present disclosure provides a computer system comprising a processor, a memory coupled to the processor, wherein the memory stores a program that, when executed by the processor, causes the processor to perform the method for obtaining a model for controlling at least one composition variable according to the present disclosure.

In another aspect, the present disclosure provides a computer system comprising a processor, a memory coupled to the processor, wherein the memory stores a program that, when executed by the processor, causes the processor to perform the method for controlling at least one composition variable

In another aspect, the present disclosure provides a non-transitory computer-readable medium storing a program that, when executed by processor, causes the processor to perform the method for controlling at least one composition variable.

In another aspect, the present disclosure provides a non-transitory computer-readable medium storing a program that, when executed by a processor, causes the processor to perform the method for obtaining a model for controlling at least one composition variable according to the present disclosure.

In another aspect, the present disclosure provides a computer program product comprising computer program code that, when executed by a processor, causes the processor to perform the method for controlling at least one composition variable according to the present disclosure.

### Example 1

In a urea plant operated by Yara International, a model to estimate the NH₃/CO₂ and H₂O/CO₂ ratios at the liquid composition outlet of a carbamate condenser was developed. Figure 1 shows the system **1** that was used for developing the model. The system **1** comprises a carbamate condenser **2** and an absorber **3.** The carbamate condenser **2** is configured to receive a gaseous stream comprising ammonia and carbon dioxide via line **4.** Inert gas, such as nitrogen, is injected into the gaseous stream comprising ammonia and carbon dioxide, via line **5.** In the carbamate condenser **2,** the gaseous stream comprising ammonia and carbon dioxide is cooled down, for example with cooling water, to produce an aqueous solution comprising ammonium salts, such as ammonium carbamate, ammonium carbonate, and / or ammonium bicarbonate. The condensation of the gaseous stream is usually only partial, and a gaseous stream depleted in ammonia and carbon dioxide exits the carbamate condenser **2** via line **6** and enters the absorber **3.** In the absorber **3**, the gaseous stream is contacted with an aqueous solution, in particular an aqueous solution comprising ammonium salts, such as ammonium carbamate, provided via line **7**. Upon contact of the gaseous stream and the aqueous solution, some of the gaseous ammonia and carbon dioxide are dissolved in the aqueous solution, which is directed back to the carbamate condenser **2**, and mixed with the condensate produced therein. An aqueous solution comprising ammonium salts is recovered from a liquid outlet via line **12**. The absorber **3** comprises a gas outlet from which uncondensed and inert gases can leave the absorber **2** via line **9**. Line **9** comprises a pressure control valve **10** that can be used to control the pressure inside the absorber **3**. The flow of aqueous solution into absorber **3** is controlled by a flow control valve **8**. The injection of an aqueous solution to the carbamate condenser **2** is necessary to avoid crystallization of the ammonium salts, in particular ammonium carbamate, therein. On the other hand, injection of aqueous solution should also be kept as low as possible: the aqueous solution recovered from line **12** is sent back to the high pressure section of the plant, for example to the urea reactor, wherein an excess of water has a negative effect on the formation of urea from ammonia and carbon dioxide. So, knowing the water content of the aqueous solution in line **12** in real time allows the operator to keep the input of aqueous solution via line **7** as optimal as possible. However, no online sensor is able to directly measure this water content, so offline laboratory analyses are required, but impractical because they take time.

Therefore, a model to estimate the NH₃/CO₂ and H₂O/CO₂ ratios at the liquid outlet of a carbamate condenser was developed.

Over a first period of time (over 18 months) during the urea production process, a plurality of online measurement data relating to a plurality of process variables, such as the plant load, the flow of gas in line **5**, the pressure and temperature inside the carbamate condenser **2**, the valve flow coefficient and opening degree of valves **8** and **10**, the temperature of the aqueous solution in line **11**, and the flow of aqueous solution in line **12**, and the density and viscosity of the aqueous solution in line **12** were measured by means of a plurality of sensors. In that first period of time, samples of the aqueous solution in line **12** were collected and the water, ammonia, and carbon dioxide contents thereof were measured using standard analytical methods, for example using an acid-based titration technique.

The two sets of data were statistically analysed and a linear regression model to predict the NH₃/CO₂ and H₂O/CO₂ ratios was obtained. The model achieved a correct estimation of all the ratios measured during the first period of time within a 3% error margin.

## Claims

1. A virtual sensing method for controlling at least one composition variable during a urea production process in a urea-producing plant, based on a plurality of online measured process variables and the viscosity of one or more liquid streams, and a model,
wherein the model is used to estimate, during the urea production process, the at least one composition variable, on the basis of the plurality of online measured process variables and the viscosity of one or more liquid streams, wherein the method includes modifying at least one of the plurality of online measured process variables for ensuring that the value of the at least one composition variable is within a predetermined range,
wherein the model is obtained by:
retrieving, over a first period of time during the urea production process, a plurality of online measurement data relating to a plurality of predetermined process variables and the viscosity of one or more liquid streams by means of a plurality of sensors arranged in the urea-producing plant, the plurality of predetermined process variables comprising at least one of the group consisting of a flow rate, a liquid level, a temperature, and a pressure;
retrieving, at time points within the first period of time, a plurality of offline measurement data of the at least one composition variable; and
processing the plurality of online and offline measurement data and performing a statistical analysis for identifying the model for predicting the at least one composition variable on the basis of the plurality of predetermined process variables.

2. The method according to claim 1, wherein the plurality of online measured process variables and viscosity values obtained by means of online measurements over a second period of time, different from the first period of time, are provided as inputs to the identified model, wherein the model provides as an output at least one predicted composition variable, which is being controlled.

3. The method according to claim 1 or 2, wherein online measurements of process variables and viscosity are stored in a data store, and a reduced data set is obtained from the data store, wherein the model is identified based on the reduced data set, the model providing a correlation between the reduced data set and the at least one composition variable.

4. The method according to any one of the preceding claims, wherein the composition variable is selected from the group consisting of a NH₃/CO₂ ratio defined as a ratio between a total equivalent NH₃ and a total equivalent CO₂, a H₂O/CO₂ ratio defined as a ratio between a total equivalent H₂O and a total equivalent CO₂, and/or an extent of reaction (or carbon dioxide conversion) defined as a ratio between urea and total equivalent CO₂.

5. The method according to any one of the preceding claims, wherein the model is used to estimate, during the urea production process, the at least one composition variable, on the basis of the plurality of online measured process variables, the viscosity of one or more liquid streams, and the density of one or more liquid streams.

6. The method according to any one of the preceding claims, wherein the process variables comprise at least one selected from the group consisting of a plant load, a CO₂ feed flow, a CO₂ flow to a CO₂ stripper, a passivation air flow to a reactor, a passivation air flow to a stripper, a carbamate recycle flow to a carbamate condenser, a carbamate recycle flow to the high-pressure scrubber, a steam flow from carbamate condenser, a total flow of NH₃, a flow of NH₃ to a carbamate condenser, a flow of NH₃ to a carbamate ejector, a flow of NH₃ to a reactor, a steam consumption of a stripper a pressure in a reactor, a carbamate condenser steam pressure, a stripper vapor exit temperature, a stripper liquid exit temperature, a temperature of an NH₃ feed, temperature of a stream comprising ammonium carbamate, a temperature in a reactor, a temperature of a urea solution from a reactor, a temperature at a liquid outlet of a stripper, a temperature at the liquid outlet of a carbamate condenser, a pressure difference in a urea reactor outlet valve, a liquid level in a reactor, a liquid level in a scrubber, a flow of inert gas to a condenser, an opening degree of a pressure valve, and a liquid level in a liquid/vapor separator.

7. The method according to any one of the preceding claims, the plurality of online measured process variables comprises a plant load, a flow of inert gas to a condenser, an opening degree of a pressure valve, a temperature at an inlet of a carbamate condenser, a pressure in a carbamate condenser, a temperature in a carbamate condenser, and Na pressure at the outlet of an absorber.

8. The method according to any one of the preceding claims, wherein the statistical analysis comprises an algorithm for performing a principal component analysis or a partial least squares analysis.

9. A method for obtaining a model for controlling at least one composition variable in a urea production process in a urea-producing plant, the method comprising:
retrieving, over a first period of time during the urea production process, a plurality of online measurement data relating to a plurality of predetermined process variables and to the viscosity of a liquid by means of a plurality of sensors arranged in the urea-producing plant, the plurality of predetermined process variables comprising at least one from the group consisting of a flow rate, a liquid level, a temperature, and a pressure;
retrieving, at time points within the first period of time, a plurality of offline measurement data of at least one composition variable; and
processing the plurality of online and offline measurement data and performing a statistical analysis for identifying the model for predicting the at least one composition variable on the basis of the plurality of predetermined process variables and the viscosity.

10. The method according to claim 10, further comprising performing simulated annealing for identifying a selection of process variables used with the model, wherein the simulated annealing comprises determining a plurality of variable sets with different combinations of process variables and viscosities, and evaluating, for each of the plurality of the variable sets, a quality of prediction of the at least one composition variable, wherein the variable set providing the highest quality of prediction of the at least one composition variable is selected for use with the model.

11. A system including a controller configured for controlling at least one composition variable in a urea production process in a urea-producing plant, based on a plurality of online measured process variables and the viscosity of one or more liquid streams, and a model, wherein the controller is configured to use the model for estimating, during the urea production process, the at least one composition variable on the basis of the plurality of online measured process variables and the viscosity of one or more liquid streams, for ensuring that the value of the at least one composition variable is within a predetermined range, wherein the model is obtainable or obtained by:
retrieving, over a first period of time during the urea production process, a plurality of online measurement data relating to a plurality of predetermined process variables and the viscosity of one or more liquid streams by means of a plurality of sensors arranged in the urea-producing plant, the plurality of predetermined process variables comprising at least one of the group consisting of a flow rate, a liquid level, a temperature, and a pressure;
retrieving, at time point within the first period of time, a plurality of offline measurement data of the at least one composition variable; and
processing the plurality of online and offline measurement data and performing a statistical analysis for identifying the model for predicting the at least one composition variable on the basis of the plurality of predetermined process variables and the viscosity of one or more liquid streams.

12. A system for obtaining a model for controlling at least one composition variable in a urea production process, the system comprising a controller configured to perform:
retrieving, over a first period of time during the urea production process, a plurality of online measurement data relating to a plurality of predetermined process variables and the viscosity of one or more liquid streams by means of a plurality of sensors arranged in the urea-producing plant, the plurality of predetermined process variables comprising at least one of the group consisting of a flow rate, a liquid level, a temperature, and a pressure;
retrieving, at time points within the first period of time, a plurality of offline measurement data of the at least one composition variable; and
processing the plurality of online and offline measurement data and performing a statistical analysis for identifying the model for predicting the at least one composition variable on the basis of the plurality of predetermined process variables and the viscosity of one or more liquid streams.

13. A computer system comprising a processor, a memory coupled to the processor, wherein the memory stores a computer program that, when executed by the processor, causes the processor to perform the method according to any one of the claims 1 to 11.

14. A non-transitory computer-readable medium storing a program that, when executed by a processor, causes the processor to perform the method according to any one of the claims 1 to 11.

15. A computer program product comprising computer program code that, when executed by a processor, causes the processor to perform the method according to any one of the claims 1 to 11.
